# EUROPEAN PATENT APPLICATION

(11) **EP 3 441 100 A1**
(43) Date of publication of application: **13.02.2019**
(21) Application number: 17185742.8
(22) Date of filing: 10.08.2017
(51) Int. Cl.: A61M 16/00, A62B 18/00, A61M 16/06

(54) **A MASK-BASED BREATHING APPARATUS AND CONTROL METHOD**

(71) Applicant: Koninklijke Philips N.V., 5656 AE Eindhoven (NL)
(72) Inventor: VAN DER SLUIS, Paul, 5656 AE Eindhoven (NL); CHEN, Shuang, 5656 AE Eindhoven (NL); KAUWENBERG, Werner Daniel Petrus, 5656 AE Eindhoven (NL); BOUMA, Peter Hermanus, 5656 AE Eindhoven (NL)
(74) Representative: de Haan, Poul Erik

(57) **Abstract**

The invention provides a breathing assistance mask. In one aspect, a pressure monitoring system is implemented. The rotation speed of a fan is used to derive a pressure or detect pressure change in the mask air chamber coupled to the fan. This avoids the need for a separate pressure sensor. The pressure across the mask is used to determine if the mask is being worn. In another aspect, signals generated with the fan motor which result from mechanical movement of the fan when it is turned off, caused by breathing of the user, are detected, and used to switch the fan on.

## Description

### FIELD OF THE INVENTION

This invention relates to a breathing apparatus in the form of a mask, for providing filtered air to the wearer of the breathing apparatus, with the flow assisted by a fan.

### BACKGROUND OF THE INVENTION

The World Health Organization (WHO) estimates that 4 million people die from air pollution every year. Part of this problem is the outdoor air quality in cities. The worst in class are Indian cities like Delhi that have an annual pollution level more than 10 times the recommended level. Well known is Beijing with an annual average 8.5 times the recommended safe levels. However, even in European cities like London, Paris and Berlin, the levels are higher than recommended by the WHO.

Since this problem will not improve significantly on a short time scale, the only way to deal with this problem is to wear a mask which provides cleaner air by filtration. To improve comfort and effectiveness one or two fans can be added to the mask. These fans are switched on during use and are typically used at a constant voltage. For efficiency and longevity reasons these are normally electrically commutated brushless DC fans.

The benefit to the wearer of using a powered mask is that the lungs are relieved of the slight strain caused by inhalation against the resistance of the filters in a conventional non-powered mask.

Furthermore, in a conventional non-powered mask, inhalation also causes a slight negative pressure within the mask which leads to leakage of the contaminants into the mask, which leakage could prove dangerous if these are toxic substances. A powered mask delivers a steady stream of air to the face and may for example provide a slight positive pressure, which may be determined by the resistance of an exhale valve, to ensure that any leakage is outward rather than inward.

There are several advantages if the fan operation or speed is regulated. This can be used to improve comfort by more appropriate ventilation during the inhalation and exhalation sequence or it can be used to improve the electrical efficiency. The latter translates into longer battery life or increased ventilation. Both of these aspects need improvement in current designs.

To regulate the fan speed, the pressure inside the mask can be measured and both pressure as well as pressure variation can be used to control the fan.

For example, GB 2032284 discloses a respirator in which the pressure inside a mask is measured by a pressure sensor and the fan speed is varied in dependence on the sensor measurements.

A pressure sensor is costly so it would be desirable to provide an alternative method of monitoring pressure inside a mask. Such pressure information may be used to control a fan within a powered respirator, but it may be used as part of any other fan-based system where pressure information is desired.

Fan-operated masks are battery-operated devices, so that it is desirable to reduce power consumption to a minimum as well as keeping the cost to a minimum. One issue is that the fan may be left on when the mask is not being worn, and this results in unnecessary power consumption. It is possible to provide sensors dedicated to detecting when the mask is worn, but this increases the cost of the breathing mask.

When the putting on the mask, a user typically activates a switch to switch on the fan. This switch adds cost to the mask, takes up space and switching on is inconvenient. An automatic electronic switch-on function would avoid disadvantages. However, this normally also requires a dedicated sensor that senses the use of the mask.

It would therefore be desirable to find a lower cost solution to detecting that the mask is worn, to enable worn to not-worn transitions and/or not worn to worn transitions to be detected.

### SUMMARY OF THE INVENTION

The invention is defined by the claims.

According to examples in accordance with an aspect of the invention, there is provided a mask comprising:
an air chamber;
a filter;
a fan for drawing air from outside the air chamber into the air chamber and/or drawing air from inside the air chamber to the outside;
a means for determining a rotation speed of the fan; and
a controller which is adapted to:
   derive a pressure or detect a pressure change in the air chamber from the rotation speed of the fan; and
   determine whether the mask is worn or not based on the derived pressure or detected pressure change.

In this system, a fan speed (for a fan which drives air into the chamber and/or expels it from the chamber) is used as a proxy of pressure measurement. To measure the fan speed, the fan itself may be used so that no additional sensors are required. The chamber may be closed in normal use, so that pressure fluctuations in the gas chamber have an influence on the load conditions of the fan and hence alter the fan electrical characteristics. Similarly, the fan electrical characteristics may determine the nature of the chamber, for example its volume, and if it is an open or closed volume.

By determining if the mask is worn, the mask design enables power to be saved when the mask is not being worn, but without requiring any additional sensors. In particular, if there is no detected pressure differential across the mask, this indicates that both sides are at atmospheric pressure and the mask is not being worn. In effect, there is no longer a closed or partially closed chamber, so that the air chamber is open to the atmosphere. The fan may be turned off if it is detected that the mask is not worn.

In one example the fan is driven by an electronically commutated brushless motor, and the means for determining rotation speed comprises an internal sensor of the motor. The internal sensor is already provided in such motors to enable rotation of the motor. The motor may even have an output port on which the internal sensor output is provided. Thus, there is a port which carries a signal suitable for determining the rotation speed.

Alternatively, the means for determining the rotation speed may comprise a circuit for detecting a ripple on the electrical supply to a motor which drives the fan. The ripple results from switching current through the motor coils, which cause induced changes in the supply voltage as a result of the finite impedance of the input voltage source.

The fan may be a two-wire fan and the circuit for detecting a ripple comprises a high pass filter. The additional circuitry needed for a motor which does not already have a suitable fan speed output can be kept to a minimum.

The mask may further comprise an outlet valve for controllably venting the air chamber to the outside. The outlet valve may comprise a passive pressure-regulated check valve or an actively driven electrically controllable valve. This may be used to make the mask more comfortable. During inhalation, by closing the valve (actively or passively), it is prevented that unfiltered air is drawn in. During exhalation, the valve is opened so that breathed out air is expelled.

The controller may be adapted to determine a respiration cycle from the pressure monitoring system, and to control the controllable valve in dependence on the phase of the respiration cycle. The pressure monitoring thus provides a simple way to determine inhalation phases, which may then be used to control the timing of a venting valve of the mask or to determine whether or not the mask is worn and hence in use.

The controller may be adapted to turn off the fan during an inhalation time. This may be used to save power. Shutting down the fan during inhalation may be desirable for a user who does not have difficulty breathing through the filter, to save power if configured in such a way.

Thus, the system may enable the mask to be operated in different modes as well as being turned off when it is not being worn.

The mask may further comprise:
a detection circuit for detecting induced current or voltage spikes caused by rotation of the fan when the fan is not being electrically driven; and
a start-up circuit for starting electrical driving of the fan in response to an output from the detection circuit,

This feature enables the fan to be started when a mask is worn, by detecting electrical spikes causes by manual rotation of the fan. This rotation is for example caused by the user wearing the mask and breathing through the fan, when the fan is not electrically driven. These movements are then detected, in order to provide automatic turn on of the fan. This approach does not require active sensing that the mask is worn but instead, the breathing of the user provides the energy for the sensing function. The sensing may be integrated into the fan circuitry with low overhead and low power consumption.

In this way, the fan may be used as a sensor for detecting transition from a worn to not worn status as well as from a not worn to a worn status of the mask.

The controller may also be adapted to determine a flow resistance of the filter. This may be used to evaluate a filter lifetime.

Examples in accordance with another aspect of the invention provide a method of controlling a mask, comprising:
drawing gas into and/or out of an air chamber of the mask using a fan;
determining a rotation speed of the fan;
deriving a pressure or detecting a pressure change in the air chamber from the rotation speed of the fan; and
determining whether the mask is worn or not based on the derived pressure or detected pressure change.

The fan may be turned off if it is detected that the mask is not worn.

The fan speed is thus used as a proxy for measurement of a pressure or relative pressure and this proxy measurement is used to detect whether or not the mask is being worn.

The method may comprise driving the fan using an electronically commutated brushless motor, and the rotation speed is determined by an internal sensor of the motor. Alternatively, the rotation speed may be obtained by detecting a ripple on the electrical supply to a motor which drives the fan. This may be applied to any type of motor, such as a conventional DC motor with brushes.

The mask may comprise an electrically controllable valve for controllably venting an air chamber to the outside. A respiration cycle may then be determined from the pressure monitoring system, and the method may comprise controlling the controllable valve in dependence on the phase of the respiration cycle. The mask may instead simply have a pressure-regulating release valve.

### BRIEF DESCRIPTION OF THE DRAWINGS

Examples of the invention will now be described in detail with reference to the accompanying drawings, in which:
Figure 1 shows a pressure monitoring system implemented as part of a face mask;
Figure 2 shows one example of the components of the pressure monitoring system;
Figure 3 shows a rotation signal during inhalation and during exhalation; and
Figure 4 shows a circuit for controlling the current through one of the stators of a brushless DC motor;
Figure 5 shows a detection circuit and start-up circuit applied to the circuit of Figure 4;
Figure 6 shows a first mask operating method; and
Figure 7 shows a second mask operating method.

### DETAILED DESCRIPTION OF THE EMBODIMENTS

The invention provides a breathing assistance mask. In one aspect, a pressure monitoring system is implemented. The rotation speed of a fan is used to derive a pressure or detect pressure change in the mask air chamber coupled to the fan. This avoids the need for a separate pressure sensor. The pressure across the mask is used to determine if the mask is being worn. In another aspect, signals generated with the fan motor which result from mechanical movement of the fan when it is turned off, caused by breathing of the user, are detected, and used to switch the fan on.

The invention is described below with reference to a breathing mask system which has two different detection functions. A first detection function (which forms the basis of this invention) is to provide pressure monitoring and use this to detect if the mask is worn or not, and in particular it enables a transition from worn to not worn to be detected. A second detection function is to enable a transition from not worn (and with the mask fan turned off) to worn to be detected.

Both detection functions aim to avoid requiring significant power consumption from any sensors, and without requiring significant additional hardware complexity.

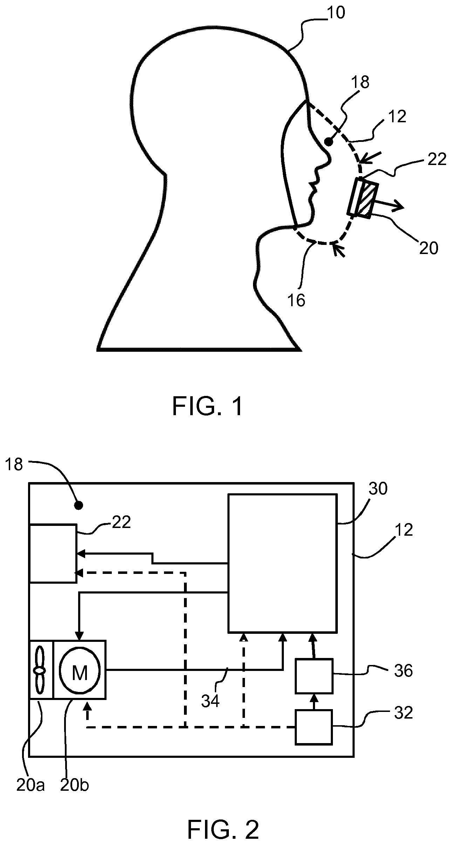

Figure 1 shows a pressure monitoring system implemented as part of a face mask.

A subject 10 is shown wearing a face mask 12 which covers the nose and mouth of the subject. The purpose of the mask is to filter air before it is breathed in the subject. For this purpose, the mask body itself acts as an air filter 16. Air is drawn in to an air chamber 18 formed by the mask by inhalation. During inhalation, an outlet valve 22 such as a check valve is closed due to the low pressure in the air chamber 18.

When the subject breathes out, air is exhausted through the outlet valve 22. This valve is opened to enable easy exhalation, but is closed during inhalation. A fan 20 assists in the removal of air through the outlet valve 22. Preferably. more air is removed than exhaled so that additional air is supplied to the face. This increases comfort due to lowering relative humidity and cooling. During inhalation, by closing the valve, it is prevented that unfiltered air is drawn in. The timing of the outlet valve 22 is thus dependent on the breathing cycle of the subject. The outlet valve may be a simple passive check valve operated by the pressure difference across the filter 16. However, it may instead be an electronically controlled valve.

There will only be a raised pressure inside the chamber if the mask is worn. In particular the chamber is closed by the face of the user. The pressure inside the closed chamber when the mask is worn will also vary as a function of the breathing cycle of the subject. When the subject breathes out, there will be a slight pressure increase and when the subject breathes in there will be a slight pressure reduction.

If the fan is driven with a constant drive level (i.e. voltage), the different prevailing pressure will manifest itself as a different load to the fan, since there is a different pressure drop across the fan. This altered load will then result in a different fan speed.

The first detection function is based in part on the recognition that the rotation speed of a fan may be used as a proxy for a measurement of pressure across the fan. It is also based in part of the recognition that pressure levels maybe used to determine whether or not the mask is worn. The invention combines these considerations to create a mask which can save power by switching off when it is not worn, and without requiring complex or costly additional sensors.

For a known pressure (e.g. atmospheric pressure) at one side of the fan, the pressure monitoring enables determination of a pressure, or at least a pressure change, on the other side of the fan. This other side is for example a closed chamber which thus has a pressure different to atmospheric pressure. However, by detecting an equal pressure on each side the fan, it can then be determined that the chamber is not closed but is connected to atmospheric pressure on both sides.

This absence of a fan speed variation may thus be used to determine that the mask is not worn and hence not in use. This information can be used to switch off the fan to save power.

The pressure monitoring system thus has a means for determining a rotation speed of the fan and a controller for deriving a pressure or detecting a pressure change from the rotation speed of the fan.

The means for determining a rotation speed may comprise an already existing output signal from the fan motor or a separate simple sensing circuit may be provided as an additional part of the fan. However, in either case fan itself is used so that no additional sensors are required.

Figure 2 shows one example of the components of the pressure monitoring system. The same components as in Figure 1 are given the same reference numbers.

In addition to the components shown in Figure 1, Figure 2 shows a controller 30, a local battery 32 and a means 36 for determining the fan rotation speed.

The fan 20 comprises a fan blade 20a and a fan motor 20b. In one example, the fan motor 20b is an electronically commutated brushless motor, and the means for determining rotation speed comprises an internal sensor of the motor. Electronically commutated brushless DC fans have internal sensors that measure the position of the rotor and switch the current through the coils in such a way that the rotor rotates. The internal sensor is thus already provided in such motors to enable feedback control of the motor speed.

The motor may have an output port on which the internal sensor output 34 is provided. Thus, there is a port which carries a signal suitable for determining the rotation speed.

Alternatively, the means for determining the rotation speed may comprise a circuit 36 for detecting a ripple on the electrical supply to the motor 20b. The ripple results from switching current through the motor coils, which cause induced changes in the supply voltage as a result of the finite impedance on the battery 32. The circuit 36 for example comprises a high pass filter so that only the signals in the frequency band of the fan rotation are processed. This provides an extremely simple additional circuit, and of much lower cost than a conventional pressure sensor.

This means the motor can be of any design, including a two-wire fan with no in-built sensor output terminal. It will also work with a DC motor with brushes.

The controller may use the rotation speed information, based on the corresponding pressure information, to determine a respiration cycle.

If the outlet valve 22 is an electronically switched value, the respiration cycle timing information may then be used to control the outlet valve 22 in dependence on the phase of the respiration cycle. The pressure monitoring thus provides a simple way to determine inhalation phases, which may then be used to control the timing of the outlet valve 22 of the mask.

In addition to controlling the outlet valve, the controller may turn off the fan during an inhalation time or an exhalation time. The controller may also turn off the fan if it is detected that the fan is not worn. This gives the mask different operating modes, which may be used to save power.

For a given drive level (i.e. voltage) the fan speed increases at lower pressure across the fan because of the reduced load on the fan blades. This gives rise to an increased flow. Thus, there is an inverse relationship between the fan speed and the pressure difference.

This inverse relationship may be obtained during a calibration process or it may be provided by the fan manufacturer. The calibration process for example involves analyzing the fan speed information over a period during which the subject is instructed to inhale and exhale regularly with normal breathing. The captured fan speed information can then be matched to the breathing cycle, from which threshold values can then be set for discriminating between inhalation and exhalation.

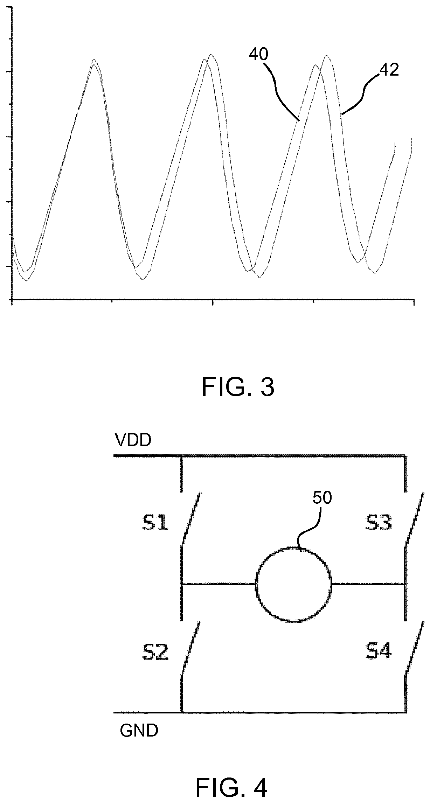

Figure 3 shows schematically the rotor position (as a measured sensor voltage) against time.

The rotational speed may be measured from the frequency of the AC component (caused by the switching events in the motor) of the DC voltage to the fan. This AC component originates from the current variation that the fan draws, imposed on the impedance of the power supply.

Figure 3 shows the signal during inhalation as plot 40 and during exhalation as plot 42. There is a frequency reduction during exhalation caused by an increased load on the fan by the increased pressure gradient. The observed frequency changes thus results from the different fan performance during the breathing cycle.

During the exhalation, fan operation forces air out of the area between face and mask. This enhances comfort because exhalation is made easier. It can also draw additional air onto the face which lowers the temperature and relative humidity. Between inhalation and exhalation, the fan operation increases comfort because fresh air is sucked into the space between the face and the mask thereby cooling that space.

During inhalation, the outlet valve is closed (either actively or passively) and the fan can be switched off to save power. This provides a mode of operation which is based on detecting the respiration cycle.

The precise timing of the inhalation and exhalation phases can be inferred from previous respiration cycles, if the fan is turned off for parts of the respiration cycle, and hence not giving pressure information.

For the fan assisted exhalation, power needs to be restored just before the exit valve opens again. This also makes sure that the next inhale-exhale cycle remains properly timed and sufficient pressure and flow are made available.

Around 30% power savings are easily achievable using this approach, resulting in prolonged battery life. Alternatively, the power to the fan can be increased by 30% for enhanced effectiveness.

With different fan and valve configurations the measurement of the fan rotation speed enables control to achieve increased comfort.

In fan configurations where the filter is in series with the fan the pressure monitoring may be used to measure the flow resistance of the filter, in particular based on the pressure drop across the fan and filter. This can be done at switch on, when the mask is not on the face for a period of time. That resistance can be used as a proxy for the age of the filter.

The first detection function as described above makes use of the fan to provide a proxy pressure measurement which is then used to detect that the mask is not worn. The pressure information may also be used for many other functions as described above. This first detection function requires the fan to be active, so it enables the transition from worn (with the fan on) to not worn to be detected. When the mask is to be worn again (or for the first time), the user may operate a manual switch to start the fan again.

However, it would be desirable to be able to switch the fan on automatically when the mask is to be worn, either the first time, or after any preceding automatic shut-down. This may be achieved using dedicated sensors, but this would require them to be permanently active, or at least making periodic sensing operations. This would re-introduce complexity to the mask and result in undesired power consumption.

The second detection function mentioned above avoids the need for a main switch or any sensors. Indeed, the fan itself is again used as a sensor. With special electronics this sensing task can be performed even when the fan is switched off.

When the mask with the fan is put on the face and the user starts to breathe, the fan will rotate even when not switched on because air is forced through the fan. The speed detection function is based on determining this rotation without the use of additional sensors with the fan switched off. That signal is subsequently used to switch on the fan for proper operation of the mask.

As mentioned above, a fan using an electronically commutated brushless DC motor has internal sensors that measure the position of the rotor and switch the current through the coils in such a way that the rotor rotates.

However, when the fan is switched off there is no longer a signal relating to the speed of rotation of the fan even if the fan is rotated mechanically.

Figure 4 shows an H-bridge circuit which functions as an inverter to generate an alternating voltage to the stator coils 50 from a DC supply VDD, GND. The inverter has a set of switches S1 to S4 to generate an alternating voltage across the coil 50.

When the fan is turned off, no electrical signal can be obtained from the power wires VDD, GND. However, electrical signals are generated due to electromagnetic induction, because when the fan is forced to rotate, the stator coil 50 moves relative to the magnets in the rotor.

These induced signals cannot be measured on the supply wires because the coils are connected to an electronic circuit which is generally deactivated when there is no driven fan rotation. Only if the electronic switches are connected in the right manner can these signals can be measured at the supply wires.

This problem can be solved by using the pulses generated directly on one of the poles of the stator coil.

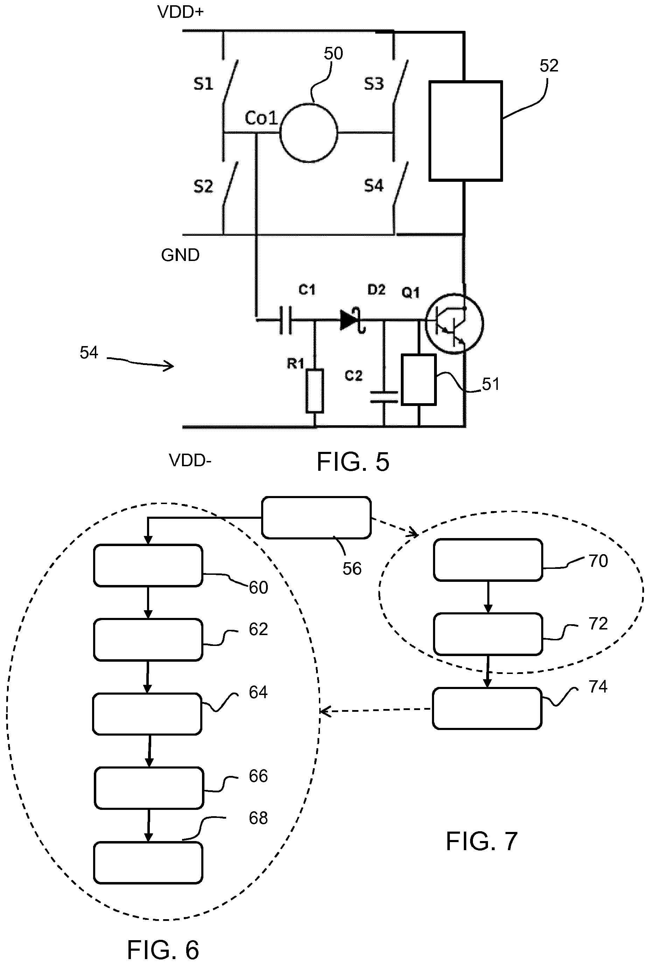

This approach is explained with reference to Figure 5.

The H-bridge circuit is provided between a high voltage rail VDD and a virtual ground. The virtual ground GND is connected to a low voltage rail VDD- through a transistor arrangement Q1.

The virtual ground may vary between VDD+ and VDD- depending on the operating state of the circuit.

The fan has a switch control circuit 52, and the fan circuitry, including the switches, coils and control circuit, are connected to VDD+ and GND as the supply voltage lines. The control circuit provides the switching signals to the switches, but to avoid cluttering Figure 5, these control signal lines are not shown. The control circuit for example include Hall sensors for rotor position sensing.

One coil terminal Col provides an output to a detection circuit 54. Since there is a DC voltage superimposed, a high pass filter of capacitor C1 and resistor R1 is used between the detection circuit 54 and the coil terminal Col. The pulses that come from the high pass filter are rectified by a diode D2 and cause charge to be stored in a storage capacitor C2.

The storage capacitor builds up a base voltage for the transistor arrangement Q1 (shown as a Darlington pair of bipolar transistors). The storage capacitor prevents the transistor arrangement quickly switching on and off in phase with the pulses.

Once enough charge is stored on the capacitor C2, the transistor arrangement Q1 will turn on (creating a closed circuit) and the fan will start to run because the supply voltage is then increased to the full VDD+ top VDD- voltage swing. That running generates enough pulses to keep the fan running.

This provides a very simple implementation.

To switch off the fan using the circuit of Figure 5, for example based on the detection that the mask is not worn as explained above, the base of the transistor arrangement Q1 may be driven to ground long enough to stop the fan from rotating. This may be achieved using a shut-down circuit 51 such as a transistor which discharges the capacitor C2.

For ultra-low power, the switch Q1 can be replaced with a MOSFET and optionally a gate amplifier. Digital logic circuits can be used to route the coil rotation signal and mask worn or not worn signal to the gate driver.

When the fan is off in Figure 5, all of the switches S1 to S4 are open (not actuated). There is no power supplied at all.

The pulses that charge the capacitor C2 will raise the voltage of the base of Q1 and eventually turn it ON. The level of the virtual ground GND is then pulled down to VDD-. At that moment, current can flow from VDD+ to VDD-. This give s power to the coils and the control circuit 52 of the fan that subsequently starts to run as long as there is enough voltage.

When C2 is charged and Q1 is on the shut down circuit 51 is used to discharge the capacitor C2 to stop the fan. For example, an npn transistor or a FET transistor may be used to short circuit the capacitor C2. The shorting signal may be derived from a breathing pattern. If there are no measured frequency fluctuations, the capacitor C2 is shorted to turn off the transistor arrangement, and thereby reduce the supply voltage because GND- rises back up towards the voltage VDD+.

Figure 6 shows a mask operating method for detecting a worn to not worn transition. The method may optionally start by turning on the fan automatically in step 56.

The method then comprises:
in step 60, drawing air into and/or out of the mask air chamber using the fan;
in step 62, determining a rotation speed of the fan; and
in step 64, deriving a pressure or detecting a pressure change in the air chamber from the rotation speed of the fan.
In step 68, the method comprises determining whether the mask is worn or not based on the derived pressure or pressure change. If the mask is not worn, based on the respiration cycle changes of Figure 3 not being apparent, the fan may be switched off to save power.

This implements the first detection function explained above.

The fan speed is thus used as a proxy for measurement of a pressure.

The method may comprise driving the fan using an electronically commutated brushless motor, and the rotation speed is determined by an internal sensor of the motor. Alternatively, the rotation speed may be obtained by detecting a ripple on the electrical supply to a motor which drives the fan.

The method may comprise determining a respiration cycle from the pressure monitoring system, shown as step 66. When an electrically controllable outlet valve is used, it may be controlled dependence on the phase of the respiration cycle.

Figure 7 shows a mask operating method for detecting a not worn to worn transition. The method comprises:
in step 70, detecting induced current or voltage spikes caused by rotation of the fan when the fan is not being electrically driven; and
in step 72, starting electrical driving of the fan in response to the detected induced current or voltage spikes.

The method may also include (subsequently) turning off the fan in step 74 if it is detected that the mask is not worn. This detection may be based on steps 60 to 68 of Figure 6.

Similarly, the initial step 56 in Figure 6 of turning on the fan may be performed based on the steps 70 and 72 of the method of Figure 7.

The mask may be for covering only the nose and mouth (as shown in Figure 1) or it may be a full face mask.

The example shown is a mask for filtering ambient air. However, the pressure detection may be used for a mask which delivers a breathing gas from an external supply, for example a breathing assistance device, such as a continuous positive air pressure (CPAP) system.

The mask design described above has the main air chamber formed by the filter material, through which the user breathes in air.

An alternative mask design has the filter in series with the fan as also mentioned above. In this case, the fan assists the user in drawing in air through the filter, thus reducing the breathing effort for the user. An outlet valve enables breathed out air to be expelled and an inlet valve may be provided at the inlet.

The invention may again be applied for detecting the pressure variations caused by breathing for controlling the inlet valve and/or the outlet valve. The fan in this example needs to be turned on during inhalation, to assist the user in drawing air through the series filter, but it may be turned off during exhalation when the outlet valve is open. Thus, the pressure information derived may again be used to control the fan to save power when the fan operation is not needed. The detection of whether the mask is worn or not may also be implemented.

It will be seen that the invention may be applied to many different mask designs, with fan-assisted inhalation or exhalation, and with an air chamber formed by a filter membrane or with a sealed hermetic air chamber.

The detection of the respiration cycle is a preferred feature as an additional use of the pressure monitoring capability, but it is optional.

As discussed above, embodiments make use of a controller, which can be implemented in numerous ways, with software and/or hardware, to perform the various functions required. A processor is one example of a controller which employs one or more microprocessors that maybe programmed using software (e.g., microcode) to perform the required functions. A controller may however be implemented with or without employing a processor, and also maybe implemented as a combination of dedicated hardware to perform some functions and a processor (e.g., one or more programmed microprocessors and associated circuitry) to perform other functions.
Examples of controller components that may be employed in various embodiments of the present disclosure include, but are not limited to, conventional microprocessors, application specific integrated circuits (ASICs), and field-programmable gate arrays (FPGAs). In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A mask comprising:
an air chamber (18);
a filter (16);
a fan (20) for drawing air from outside the air chamber (18) into the air chamber and/or drawing air from inside the air chamber to the outside;
a means (34, 36) for determining a rotation speed of the fan; and
a controller (30) which is adapted to:
derive a pressure or detect a pressure change in the air chamber from the rotation speed of the fan; and
determine whether the mask is worn or not based on the derived pressure or pressure change.

2. A mask as claimed in claim 1, wherein the controller is adapted to turn off the fan if it is determined that the mask is not worn.

3. A mask as claimed in claim 1 or 2, wherein the fan (20) is driven by an electronically commutated brushless motor, and the means for determining rotation speed comprises an internal sensor of the motor.

4. A mask as claimed in claim 1 or 2, wherein the means (36) for determining rotation speed comprises a circuit for detecting a ripple on the electrical supply to a motor which drives the fan.

5. A mask as claimed in any preceding claim, further comprising an outlet valve (22) for controllably venting the air chamber (18) to the outside, wherein the outlet valve (22) comprises a passive pressure-regulated check valve or an actively driven electrically controllable valve.

6. A mask as claimed in claim 5, wherein the controller (30) is adapted to determine a respiration cycle from the pressure monitoring system, and to control the outlet valve (22) in dependence on the phase of the respiration cycle.

7. A mask as claimed in claim 5, wherein the controller (30) is adapted to determine a respiration cycle from the pressure monitoring system, wherein the controller is adapted to turn off the fan during an inhalation time.

8. A mask as claimed in any preceding claim, further comprising:
a detection circuit for detecting induced current or voltage spikes caused by rotation of the fan when the fan is not being electrically driven; and
a start-up circuit for starting electrical driving of the fan in response to an output from the detection circuit.

9. A mask as claimed in any preceding claim, wherein the filter comprises an outer wall (16) of the air chamber, or a filter member in series with the fan.

10. A mask as claimed in any preceding claim, wherein the controller (30) is adapted to determine a flow resistance of the filter.

11. A method of controlling a mask, comprising:
(60) drawing gas into and/or out of an air chamber of the mask using a fan;
(62) determining a rotation speed of the fan;
(64) deriving a pressure or detecting a pressure change in the air chamber from the rotation speed of the fan; and
(68) determining whether the mask is worn or not based on the derived pressure or detected pressure change.

12. A method as claimed in claim 11 comprising turning off the fan if it is detected that the mask is not worn.

13. A method as claimed in claim 11 or 12, comprising:
driving the fan using an electronically commutated brushless motor, and determining the rotation speed by an internal sensor of the motor; or
driving the fan using a motor, and the determining the rotation speed by detecting a ripple on the electrical supply to the motor.

14. A method as claimed in claim 11, 12 or 13 implemented within a mask which comprises a outlet valve (22) for venting the gas chamber to the outside and the method comprises (66) determining a respiration cycle from the derived pressure or detected pressure change, and controlling the outlet valve and/or the controlling the fan in dependence on the phase of the respiration cycle.

15. A method as claimed in any one of claims 11 to 13 further comprising:
(70) detecting induced current or voltage spikes caused by rotation of the fan when the fan is not being electrically driven; and
(72) starting electrical driving of the fan in response to the detected induced current or voltage spikes.
